# EUROPEAN PATENT APPLICATION

(11) **EP 1 745 741 A1**
(43) Date of publication of application: **24.01.2007**
(21) Application number: 05450122.6
(22) Date of filing: 19.07.2005
(51) Int. Cl.: A61B 5/0402, A61B 5/0404

(54) **Ruler for evaluation of signals**

(71) Applicant: Moretti-Prucher, Gabriele, 5026 Salzburg (AT)
(72) Inventor: Moretti-Prucher, Gabriele, 5026 Salzburg (AT)
(74) Representative: Babeluk, Michael

(57) **Abstract**

Ruler for evaluation of signals like cardiac signals obtained by ECG having a template (1) with at least one transparent display (2) adapted to show at least a reference line (12) and a calliper line (13) being parallel to the reference line (12). An accurate and failsafe measurement is possible by the fact that at least one input means (4, 5, 6) is provided for setting the distance between the reference line (12) and the calliper line (13) and that the calculation unit is provided for calculating at least one parameter depending on the distance of the reference line (12) to calliper line (13) which parameter is displayed on the display.

## Description

The invention relates to a ruler for evaluation of signals according to the preamble of claim 1.

Cardiac signals obtained by ECG have to be evaluated by a medical doctor. One fundamental step of nearly every evaluation is the determination of a time interval between two different events. For example, by picking up the time interval between two characteristic points of the signal the frequency can be calculated. Usually, the time interval between two events is not measured directly but indirectly since at first the signal is recorded on paper. Then, in a first step the spatial difference between the two events is measured. In a second step that distance is divided by the speed of the paper during recording so that the time interval between the events is obtained.

In practice, there are some difficulties for obtaining an exact and reliable evaluation of an ECG plot. At first the measurement of the distance often is not accurate so that the frequency obtained by the time interval differs from the real value. Another problem is that errors may occur during calculation so that the calculated values may be completely wrong.

Prior art shows some mechanical solutions for obtaining distances from the plot some of them connected with scales for conducting approximate calculations. Such solutions are shown in US 4,388,759 A or US 5,174,040 A, for example. Further electronic devices are known for direct evaluation of cardiac signals. However, such devices are limited to the certain evaluations so that it is not possible for the doctor to make individual evaluation depending on each case.

Further AU 200020723 A1 shows an electronic scale ruler which is designed for making drawings but which is not able to evaluate signals.

Is in an object of the invention to provide a ruler which avoids the above disadvantages and which is able to assist in evaluation of signals by making measurements exact and by avoiding measurement errors. Further object of the invention is to provide a ruler which is small, easy to handle and cheap in production.

The above objects are achieved by a ruler according to claim 1.

The invention concerns an electronic ruler for measuring surface and/or internal cardiac signals, which consists of a template to be put over the ECG.

The prefered dimensions are 170 mm length and 70 mm height, but it can be smaller (like a credit card) or larger, in accordance with the needs, which allow to easily hold it in a pocket.

It is provided with a transparent LCD, to execute the measurements with moving callipers; with two alphanumeric displays, to indicate parameter(s) and relevant value(s) and with three switches to select parameter and perform measurements.

It is provided with two electrodes, preferably on the rear of the ruler, to collect a real time ECG for immediate analysis. The electrodes are shaped as clips, to be connected to commercially available skin ECG electrodes, in case of dry skin or low signal amplitude.

The power supply is given by solar cells (preferably), or batteries (rechargeable or not), or by a combination of the two, solar cells and batteries, and it is controlled by a microprocessor.

It is provided with a beeper to acoustically indicate the current operational task, like change of parameter, movement of cursors and QRS detections. The beeper can be switched ON, at different acoustic levels, or OFF.

The invention relates to an electronic ruler with a transparent LCD, which acts as measuring devices by means of movable callipers, two alphanumeric displays, which indicates the parameter(s) and the associated value(s), an integrated microprocessor and three switches for the selection of the parameter and the execution of the measurement. The LCD-display may be in color or in black and white.

To evaluate a surface ECG, it is required to perform several accurate measurements on the printed trace, mainly based on time intervals and signal amplitudes.

The most important intervals are:
- P wave: duration of the atrial depolarization
- PQ interval: time between the beginning of the atrial depolarization to the beginning of ventricular depolarization
- QRS complex: duration of the ventricular depolarization
- QT interval: time between the beginning of the ventricular depolarization and the end of the ventricular repolarization
- QTc: corrected QT after the formula: QTc=QT/squared RR
- RR interval: time between two consecutives ventricular depolarizations (which allows to compute the Heart Rate: HR=60000/RR)

The most important amplitudes are:
- P wave: maximum amplitude of the atrial depolarization
- QRS complex: maximum amplitude of the ventricular depolarization (the single Q, R and S amplitudes of two different leads allow to compute the mean ventricular axis)
- ST elevation: maximum amplitude of the segment between the end of the ventricular depolarization till the beginning of the ventricular re-polarization
- T wave: maximum amplitude of the ventricular repolarization

In case of an endocardial recording, the most important measurements are the delays between events; for instance, one event can be the local depolarization of the cardiac tissue (spontaneous or induced) or an electrical signal delivered to the heart.

In all cases, the measurement of intervals is an indirect measurement, since that it is the distance which is measured and then divided by the speed of the paper: time interval = distance/speed ([s]=[mm]/[mm/s]).

Since different speeds are used (5 - 10 - 12,5 - 25 - 50 - 100 - 200 - 400 mm/s), the manual evaluation of the time intervals can take long time and generate errors. The calculation of the heart rate, of the QTc and of the mean ventricular axis is not immediate, and requires some extra time.

Further the invention relates to a method according to claim 10.

In the following the invention will be explained by preferred and embodiments as shown in the enclosed drawings. The drawings show:
- Fig. 1: first embodiment of the ruler of the invention;
- Fig. 2: the ruler of Fig. 1 applied over an ECG-plot;
- Fig. 3: to Fig. 18 the ruler of Fig. 1 in different status; and
- Fig. 19: shows a diagram explaining the calculation of Mean QRS Axis<.

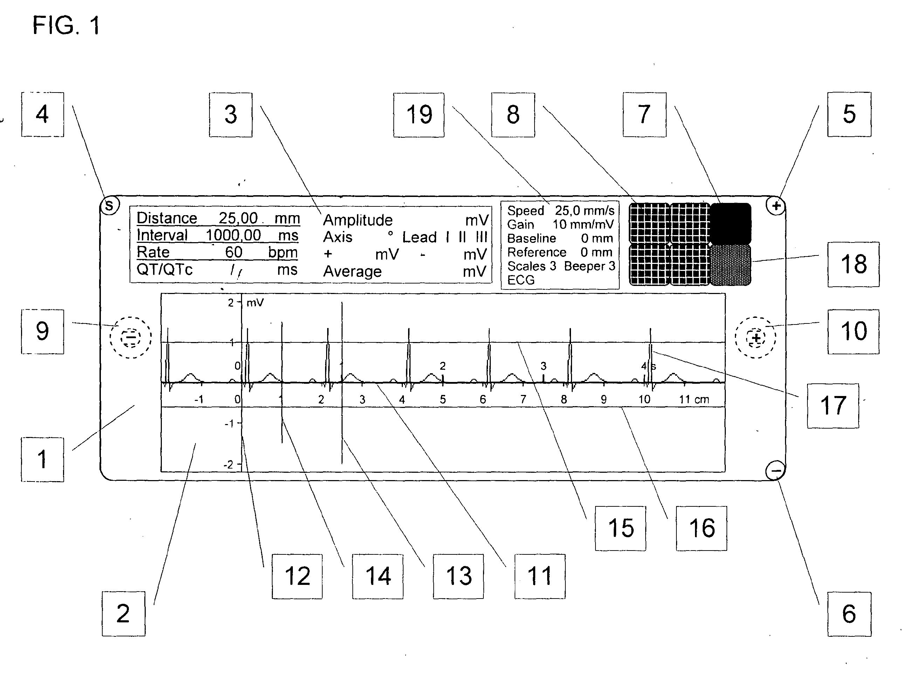

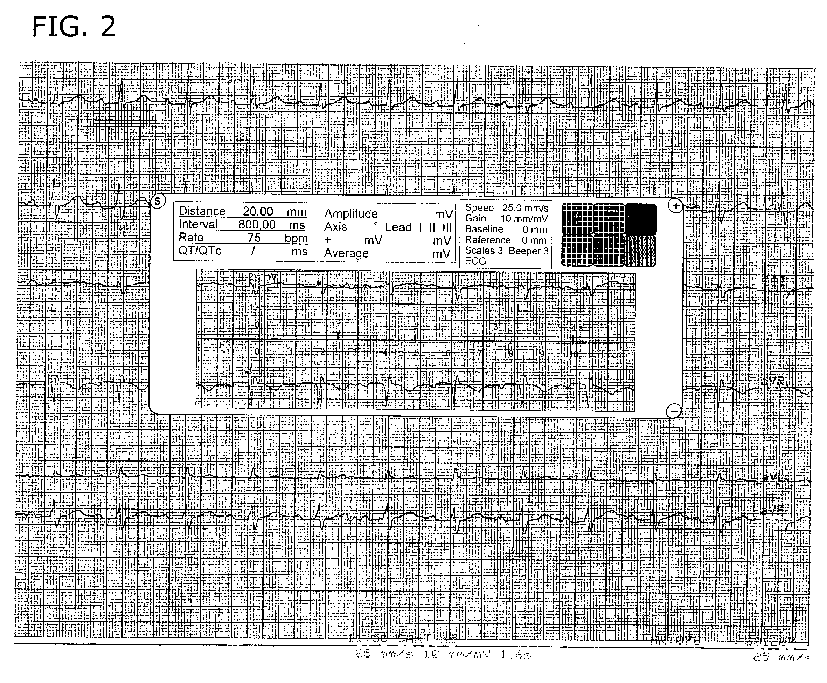

The ruler of fig. 1 consists of a template 1 preferably made of flexible plastic formed to a rectangle of the dimension of 170 x 70 mm. Integrated in the template 1 is a LCD-display 2 which is transparent. Preferably the horizontal and vertical resolution of the display is 4 dots per mm. Above the LCD-display 2 a second LCD-display 3 and a third LCD-display 19 (setup display) are provided which may be also transparent but which may be also shaded or opaque. A switch 4 in the upper left corner of template 1 allows the selection of the measuring display 3 or of third (setup) display 19 and inside the selected display it allows to scrolling of the parameters for measuring or setting up. Two switches 5 and 6 are provided in the upper right and lower right corner of template 1 for moving the lines of display 2 for changing the values of setting up parameters of third display 19 and for ECG recording as it is described in the following. All three displays 2, 3 and 19 can be of "touch screen"-type so that all functions can be selected by pointing directly on the displays 2, 3 and 19 by a stylus or by fingers. Alternatively, switches 4, 5 and 6 are preferably of "sensitive"-type, i.e. they allow activation by simply touching with fingers without any mechanical movement. They may also be activated by a lateral touch along the 2 mm border of the ruler.

A microprocessor 7 which is mounted on the template 1 controls the ruler. One or more solar cells 8 (eventually combined with rechargeable batteries) are provided for the power supply. Two electrodes 9 and 10 serve for the input of a real time ECG. A beeper 18 indicates the current operational task, like change of display, change of parameter, movement of cursors and QRS detections.

On display 2 a horizontal base line 11 is displayed. Parallel to base line 11 are an upper amplitude line 15 and a lower amplitude line 16. The distance of upper amplitude line 15 and lower amplitude line 16 from base line 11 may be varied by pressing switches 5 or 6. Further in the left region of display 2 a vertical reference line 12 is displayed. Parallel to reference line 12 a calliper line 13 is provided wherein the distance between lines 12 and 13 may be varied by pressing switches 5 and 6. Between reference line 12 and calliper line 13 an intermediate line 14 is displayed which may be moved either independent from calliper line 13 or in a certain relation to the movement of the line 13.

In the following the function of the ruler of the invention is explained.

Selection switch 4 is used to choose the display (e.g. by keeping it pressed for more than 3 seconds) and to choose parameter (by keeping it pressed for less than 3 seconds), in the selected display, which appears highlighted (e.g. underlined or blinking), between the following steps:
for LCD 3:
   - Distance, Interval and Rate
   - QT/QTc
   - Amplitude+ or Amplitude- or Amplitude
   - Axis Lead I + mV or Axis Lead I - mV or Axis Lead II + mV or Axis Lead II - mV or Axis Lead III + mV or Axis Lead III - mV
   - Average mV
for LCD 19:
   - Speed
   - Gain
   - Baseline
   - Reference
   - Scales
   - Beeper
   - ECG

Each time switch 4 is pressed longer than 3 seconds, the present active display is deselected and the other one is selected.

Each time switch 4 is pressed for less than 3 seconds, the current parameter of the active display is deselected and the next parameter of the same display is selected. If the current parameter is the last of the list, the next will be the first of the list.

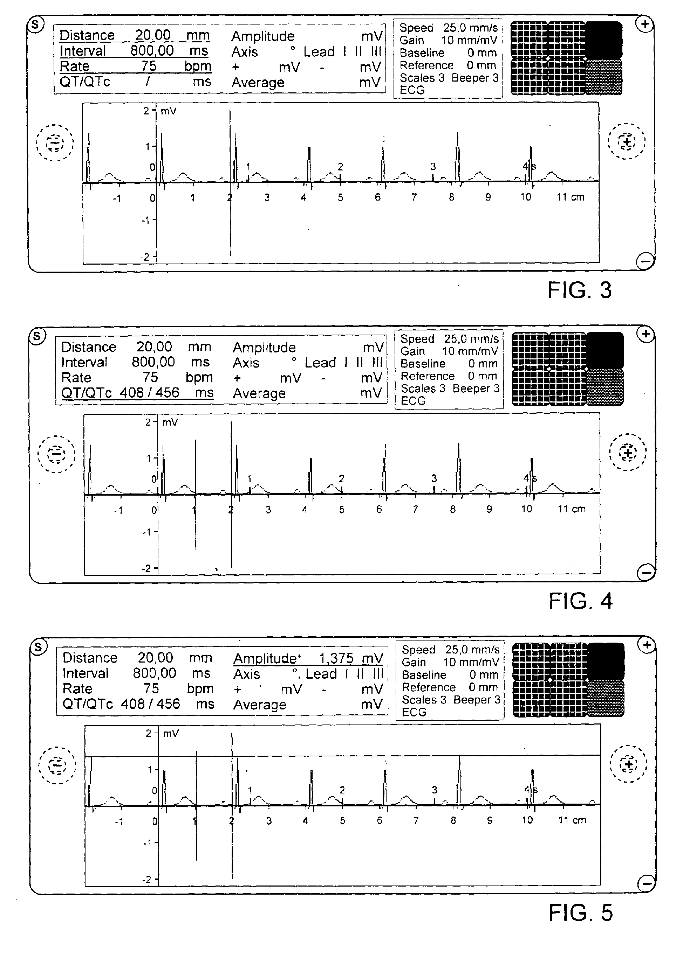

Fig. 2 shows the use of the ruler by applying it upon a plot of an ECG.

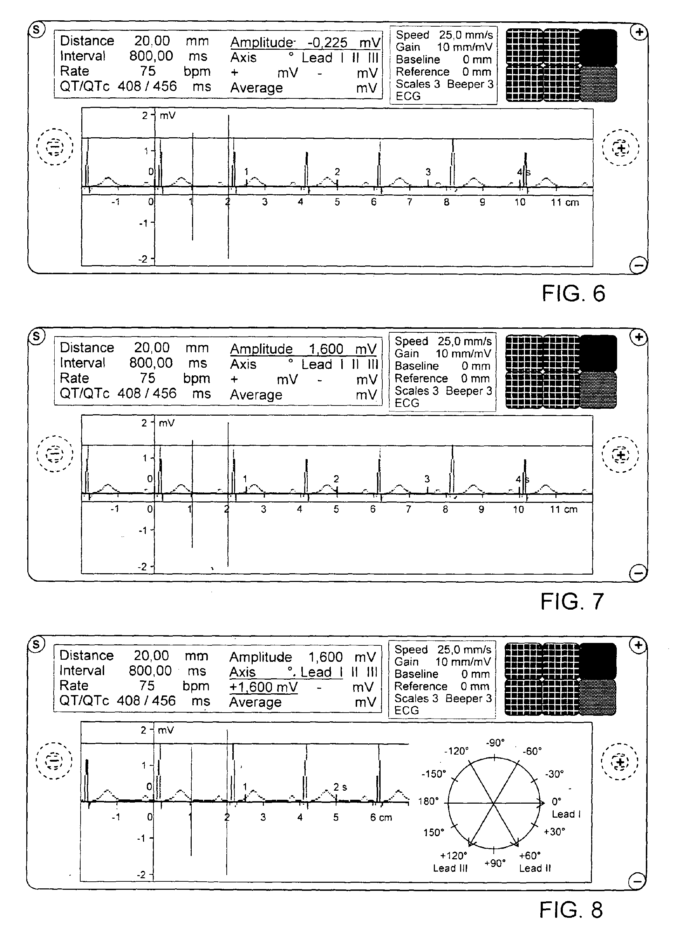

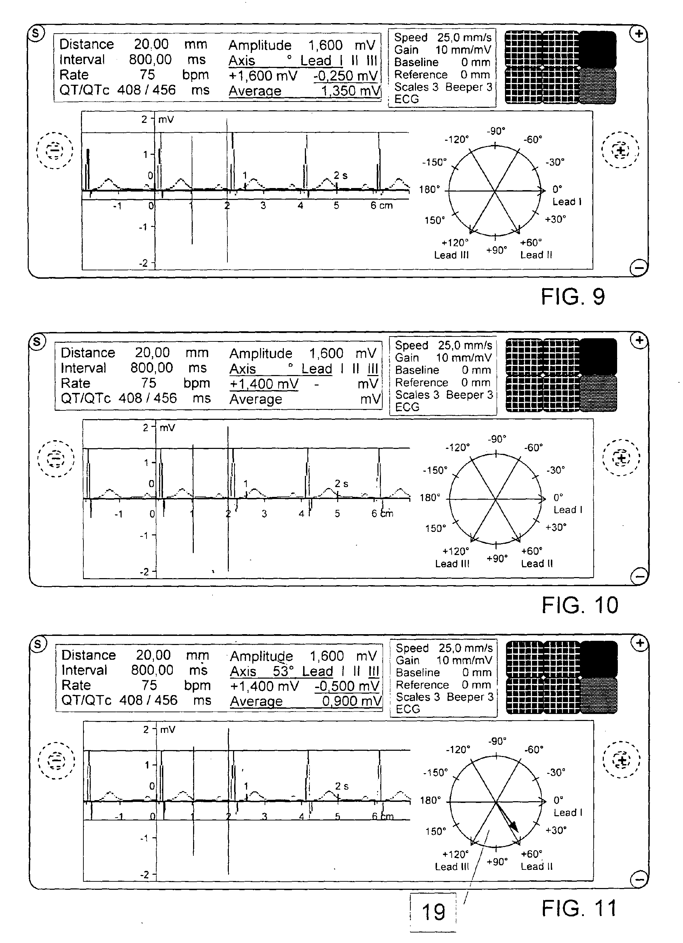

For LCD 3:
- Distance, Interval and Rate, all three highlighted together and activated by default once the ruler is turned ON. Default value for distance is 25 mm, which corresponds to an Interval of 1000 ms and to a rate of 60 bpm. After having put the ruler on the ECG with base line 11 in correspondence of the base line 11 of the ECG, and reference line 12 in correspondence of the beginning of the interval to be measured (e.g. the beginning of the complex QRS), the Calliper 13 is moved right and left by pressing switches 5 and 6, and the values of Distance, Interval and Rate are immediately updated on the display 3. Fig. 3.
- QT/QTc: once highlighted, line 14 turns on at 10 mm from Reference 12 as default. It is moved right and left by pressing switches 5 and 6, and the values of QT and QTc are immediately updated on the display 3. Fig. 4.
- Amplitude+: once highlighted, line 15 turns on at 10 mm, as default, over base line 11. It is moved up and down by pressing switches 5 and 6, and the value of the amplitude from base line 11 and Amplitude+ is immediately updated. The default gain is 10 mm/mV. It can be set to 5, 10 or 20 mm/mV in parameter Gain of third display 19. Fig 5.
- Amplitude-: once highlighted, line 16 turns on at 5 mm, as default, under base line 11. It is moved up and down by pressing switches 5 and 6, and the value of the amplitude from base line 11 and line 16 is immediately updated. The default gain is 10 mm/mV. It can be set to 5, 10 or 20 mm/mV in parameter Gain of third display 19. Fig. 6.
- Amplitude: the amplitude from line 16 to line 15 is displayed. The default gain is 10 mm/mV. It can be set to 5, 10 or 20 mm/mV in parameter Gain of third display 19. Fig. 7.
- Axis Lead I, + mV: Amplitude+ of Lead I is measured by moving line 15 by pressing switches 5 and 6. Fig. 8. Once axis is selected baseline 11 lines 15 and 16 become shorter to allow graph 19 to be displayed
- Axis Lead I, - mV: Amplitude- of Lead I is measured by moving line 16 by pressing switches 5 and 6. Fig. 9.
- Average: the sum of Amplitude+ and Amplitude- of Lead I is shown. Fig. 9.
- Axis Lead II, + mV: Amplitude+ of Lead II is measured by moving line 15 by pressing switches 5 and 6.
- Axis Lead II, - mV: Amplitude- of Lead II is measured by moving line 16 by pressing switches 5 and 6.
- Average: the sum of Amplitude+ and Amplitude- of Lead II is shown.
- Axis Lead III, + mV: Amplitude+ of Lead III is measured by moving line 15 by pressing switches 5 and 6. Fig. 10.
- Axis Lead III, - mV: Amplitude- of Lead III is measured by moving line 16 by pressing switches 5 and 6. Fig. 11.
- Average: the sum of Amplitude+ and Amplitude- of Lead III is shown. Fig. 11.

If at least two Leads are measured, the value of the mean QRS axis is computed by the microprocessor and displayed on LCD 3 together with the relevant graph. Fig.11. If all three Leads are measured, the average of the three values (Leads I and II, Leads I and III and Leads II and III) of mean QRS axis is computed by the microprocessor and displayed on LCD 3.

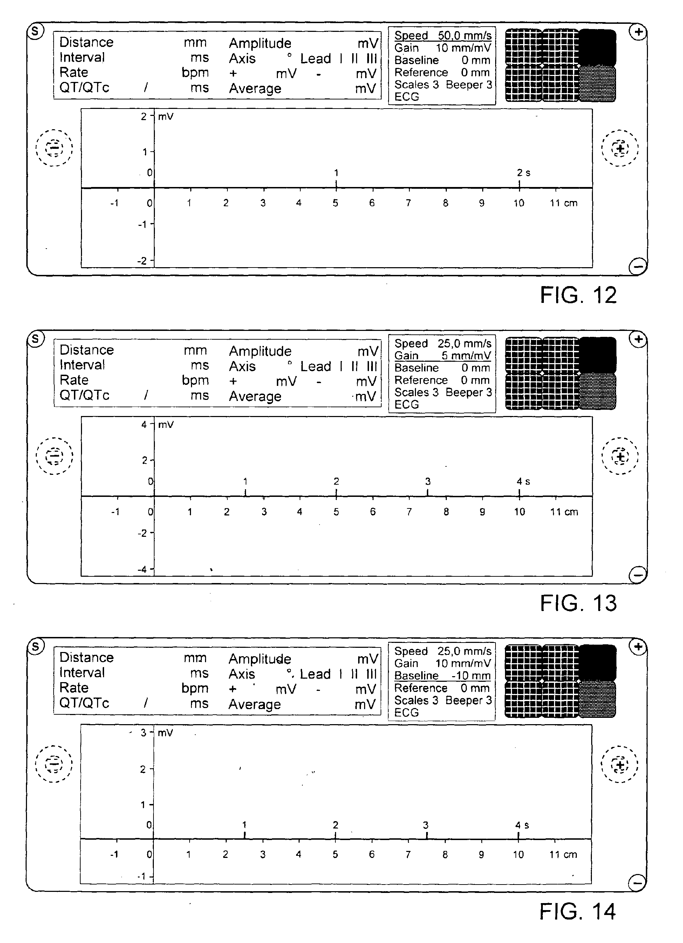

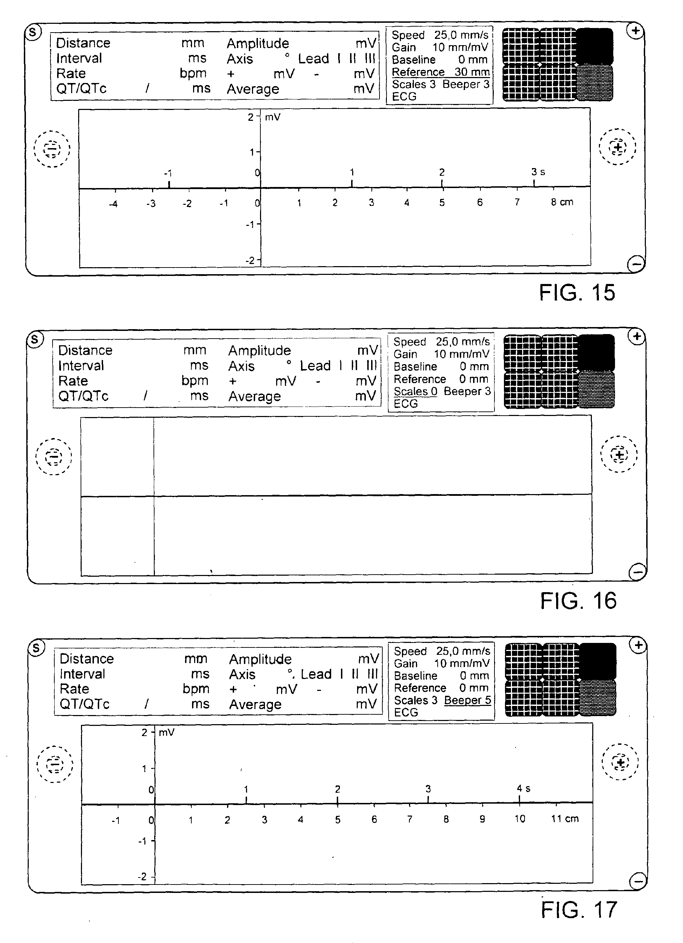

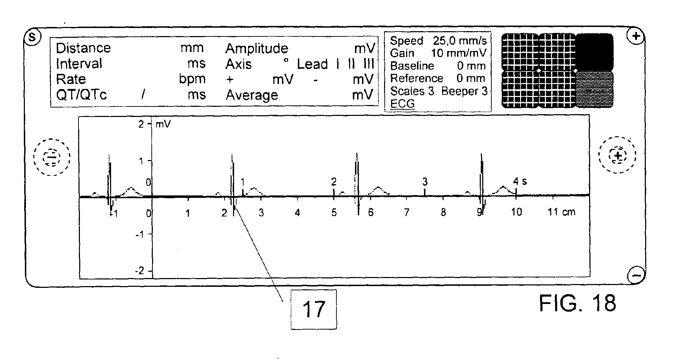

For LCD 19:
- Speed: the default value is 25 mm/s. It is possible to choose between 5 - 10 - 12,5 - 25 - 50 - 100 - 200 - 400 mm/s by means of switches 5, to increase, and 6 to decrease the present value. Fig. 12. The markers of the seconds on Baseline 11 are automatically updated to the new chart speed. If the displays are "touch screen", every time the parameter Speed is touched, its content increases to the next value of the list. If the current value is the last of the list, it restart from the first value of the list.
- Gain: it is the multiplier factor of the vertical resolution. Default value is 10 mm/mV. It can be programmed to 5, 10 or 20 mm/mV. Fig. 13. If the displays are "touch screen", every time the parameter Gain is touched, its content increases to the next value of the list. If the current value is the last of the list, it restart from the first value of the list.
- Baseline: Baseline 11 is set for default in the center of the vertical dimension of display 2. Once parameter Baseline is activated, it is movable up and down by pressing switches 5 and 6 in case of need. Fig. 14.
- Reference: Reference line 12 is set for default at 35 mm from the left border of the Ruler, to allow an easy placement of the left thumb on it. However, it can be adjusted right or left, by pressing switches 5 and 6 once parameter Reference is selected. Fig. 15. This is particularly useful after having recorded an ECG.
- Scales: Horizontal and/or vertical scales may be deactivated or activated by selecting 0, 1, 2 or 3 in Fig. 16.
- Beeper: once highlighted, the level of the beeper can be selected between several values, or switched OFF. By pressing switch 5, to increase the level and by pressing switch 6 to decrease the level or to turn it OFF. Fig. 17. If the displays are "touch screen", every time the parameter Beeper is touched, its content increases to the next value of the list. If the current value is the last of the list, it restart from the first value of the list.
- ECG: after having applied the ruler on a patient, with electrodes 9 and 10 in correspondence of suitable ECG locations (e.g. electrode 9 on the right wrist and electrode 10 on the left wrist), an ECG trace 17 can be started by pressing switch 5, and recorded at the selected speed and gain. Fig. 18. Once the trace has been ended, the required parameters can be measured as per the described procedures after having selected display 3. Electrodes 9 and 10 can be used directly on the skin. However, they are shaped as clips to be connected to currently used skin electrodes, for more stable contact and in case of dry skin or low ECG signal. If the displays are "touch screen", the words Start and Delete appear beside ECG, to allow the recording of the ECG.

Designated with 17 is the graph recorded on paper obtained by ECG or acquired through electrodes 9, 10.

Fig. 3 shows the setup of the ruler for the measurement of distances, intervals and rates. In display 2 only base line 11, reference line 12 und calliper line 13 are visible. In the second display 3 the default values for interval and frequency (rate) are shown upon the speed of 25 mm/s shown in third display 19.

Fig. 4 shows the measurement of QT and QTc. Further in addition to the displayed lines of Fig. 3 intermediate line 14 is visible.

Fig. 5 shows the setup for measuring the positive displacement of the amplitude.

Fig. 6 shows an alternative setup for measuring the negative displacement of the amplitude.

Fig. 7 shows the calculation of amplitude.

Fig. 8 shows the measurement of Axis Lead I+.

Fig. 9 shows the measurement of Axis Lead I- and calculation of average for Lead I.

Fig. 10 shows the measurement of Axis Lead III+.

Fig. 11 shows the measurement of Axis Lead III- and calculation of average for Lead III. Further, the value of the mean QRS axis is shown together with the relevant graph 19.

Fig. 12 shows the set of charged speed.

Fig. 13 shows the set of scale Gain.

Fig. 14 shows the set of position of base line 11.

Fig. 15 shows the set of level of position of reference line 12.

Fig. 16 shows the set of scales.

Fig. 17 shows the set of level of beeper.

Fig. 18 shows after recording of ECG by using electrodes 9 and 10.

The present invention allows an accurate and failsafe evaluation of signals like cardiac signals.

### Now the method for using the above ruler is explained:

The ruler can be easily hold in a pocket, and because of the independent power supply, it is always ready for use. Appendix 1 shows a detailed description of the mathematical computation of all the measured parameters.

### In case of ECG reading, the operator proceeds as follows:

The ruler is put over the ECG under examination, and it goes ON automatically as soon as the solar cells are illuminated by the environmental light.

Baseline 11, Reference 12 and Calliper 13 are displayed, with Calliper set for default at 25 mm from Reference, and Distance, Interval and Rate of display 3 are activated at the values of 25 mm, 1000 ms and 60 bpm.

Default values of display 19 are: Speed 25 mm/s, Gain 10 mm/mV, Baseline 0 mm (which corresponds to the center of the height of display 2), Reference 0 mm (which corresponds to 35 mm from the left border of the ruler), Scales 3 (which corresponds to vertical as well as horizontal values and units activated) and Beeper 3 (medium lacoustic level between 5 different levels).

All these values have been chosen because are the most commonly used in practice.

In case of Distances, Intervals and Rates, the ruler is ready for measurement (the three parameters are underlined).

Eventually, the ruler is also moved up and down, so that Baseline 11 corresponds to the baseline of the ECG trace under evaluation.

This positioning can be easily done over a table as well as in standing position, keeping the ECG paper with both hands, and the ruler moved with the left thumb pressed over the left inferior corner of it.

For example, the event to be measured as first is the duration of the ventricular depolarization, the QRS complex.

Generally the recording Speed is 25 mm/s and the vertical Gain is 10 mm/mV, and the ruler is set already at that values. If different values were used during recording, the parameter Speed and the parameter Gain of display 19 have to be set to the equal values of the ECG.

Display 19 is activated by pressing switch 4 for more that 3 seconds. Once activated, the first parameter is selected, and the relevant value can be modified by switches 5 and 6. Other parameters can be selected, clicking switch 4 for less than 3 seconds, and adjusted by switches 5 and 6. Than, display 3 can be activated again, by pressing switch 4 for more than 3 seconds.

The ruler is put over the ECG (Fig. 2) and, loocking thrugh transparent display 2, it is moved left and right, so that Reference 12 corresponds to the beginning of the event under measurement, in this example the beginning of the Q wave (Fig. 3). Calliper 13 is than moved toward the end of wave S of the complex under evaluation, by switches 5, to move right, and 6, to move left. Each step of the Calliper 13 is 0,25 mm. The values of Distance, Interval and Rate are updated at each step. Once Calliper 13 is over the end of wave S, the searched value, the duration of the ventricular depolarization, is displayed after parameter Interval (for instance 120 ms).

The same procedure is used for the measurement of any other interval, like P wave, PQ interval, T wave, etc.

Continuing the example, the next to be measured is the heart rate. Keeping the ruler in the same position, Calliper 13 is moved right to the beginning of the Q wave of the next QRS complex. As soon as it is in the required position, the searched rate is shown (e.g. 75 bpm). As always, also the relevant values of Distance and Interval are displayed. Fig.3.

Next to be measured is the QT interval. By pressing switch 4 for less than 3 seconds, Distance, Interval and Rate are deselected, QT/QTc is selected (underlined) and line 14 turns ON at 10 mm right from Reference 12. By switches 5 and 6, line 14 is moved and set to the end of the T wave. QT interval is shown on display 3, as well as the relevant corrected value (e.g. 408/456 ms). Fig.4.

Now the amplitude of the QRS has to be measured. By pressing switch 4, QT/QTc is deselected, Amplitude+ is selected and line 15 turns ON at 10 mm over Baseline 11. The ruler has to be placed so that Baseline 11 corresponds to the baseline of the ECG trace under evaluation. By switches 5 and 6, line 15 is moved up and down to meet the highest point of the QRS (Fig. 5). The amplitude of this point respect to Baseline 11 is shown on display 3 (e.g. 1,375 mV).

Pressing switch 4, Amplitude- is selected, and line 16 turns ON at 5 mm under Baseline 11. By switches 5 and 6, line 16 is moved up and down to meet the lowest point of the QRS (Fig. 6). The amplitude of this point respect to Baseline 11 is shown on display 3 (e.g. -0,225 mV).

By clicking switch 4, Amplitude- is deselected, and Amplitude is selected, and the difference of the two previous values is shown (e.g. 1,600 mV), which is the total displacement of the ventricular depolarization (Fig. 7).

Finally, the ventricular mean electrical axis has to be measured. For this, two of the three standard leads (Lead I, Lead II and Lead III) have to be used. Let suppose to use Lead I and Lead III.

The ruler is put over the ECG, with Baseline 11 in correspondence of baseline of Lead I.

By switch 4, Axis Lead I+ is selected.

Baseline 11, line 15 and line 16 will be shorter to allow graph 19 to be displayed on the right side of display 2.

Line 15 is moved up and down to meet the highest point of the QRS (Fig. 8), and the relevant amplitude is shown on display 3 (e.g. 1,600 mV).

By switch 4, Axis Lead I- is selected. Line 16 is moved up and down to meet the lowest point of the QRS (Fig. 9), and the relevant amplitude is shown on display 3 (e.g. -0,250 mV).

The Average of the two is computed, by adding the two values, and displayed (e.g.: 1,350 mV)

By clicking switch 4 twice, Axis Lead III+ is selected, and, with the same procedure used for Lead I, the values of the highest (Fig. 10), lowest and Average amplitudes (Fig. 11) of Lead III are shown (e.g. 1,400 mV, -0,500 mV, 0,900 mV).

The value of the axis is computed, and displayed on display 3 (e.g.: 53°). A graph 19 of the mean electrical QRS axis is shown on display 2 (Fig. 11).

If all the three standard leads are used, the average of the three values is computed and shown.

### In case of ECG recording, the operator proceeds as follows:

By pressing for more than 3 seconds switch 4, display 19 is activated, and the first parameter (Speed) is selected.

Default value is 25 mm/s; if required, it can be changed by pressing switches 5 or 6.

Clicking switch 4 once, parameter Gain is selected. Default value is 10 mm/mV; if required, it can be changed by pressing switches 5 or 6.

Clicking switch 4 five further times, ECG is selected (underlined).

The ruler is applied over the patient, so that electrodes 9 and 10 correspond to suitable positions for ECG recording (e.g. electrode 9 on the right wrist, and electrode 10 on the left one).

In case of low signal, presence of noise or dry skin, commercially available ECG electrodes can be applied on the patient, and the ruler can be connected to them directly, since electrode 9 and 10 are shaped as standard ECG clips.

Clicking switch 5, the recording starts at the left of display 2, and proceeds till the end of the same display. Every time switch 5 is pressed, the previous recording is deleted, and a new one starts. Pressing switch 6, the recorded trace is deleted.

After having recorded the ECG 17 (Fig. 18), all the measurements previously described can be performed on the recorded trace, by selecting display 3.

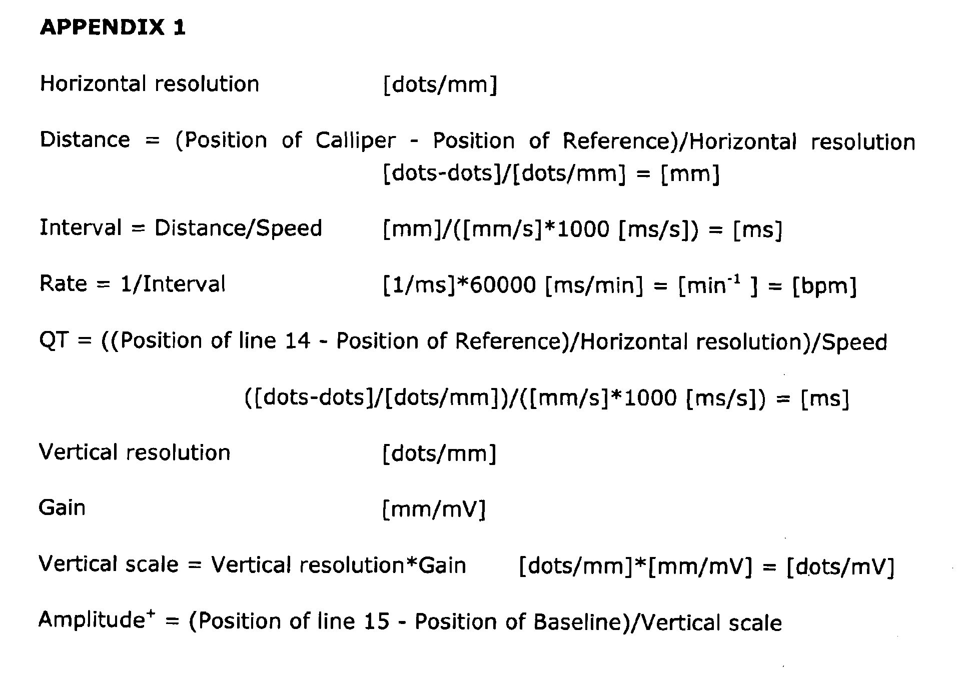

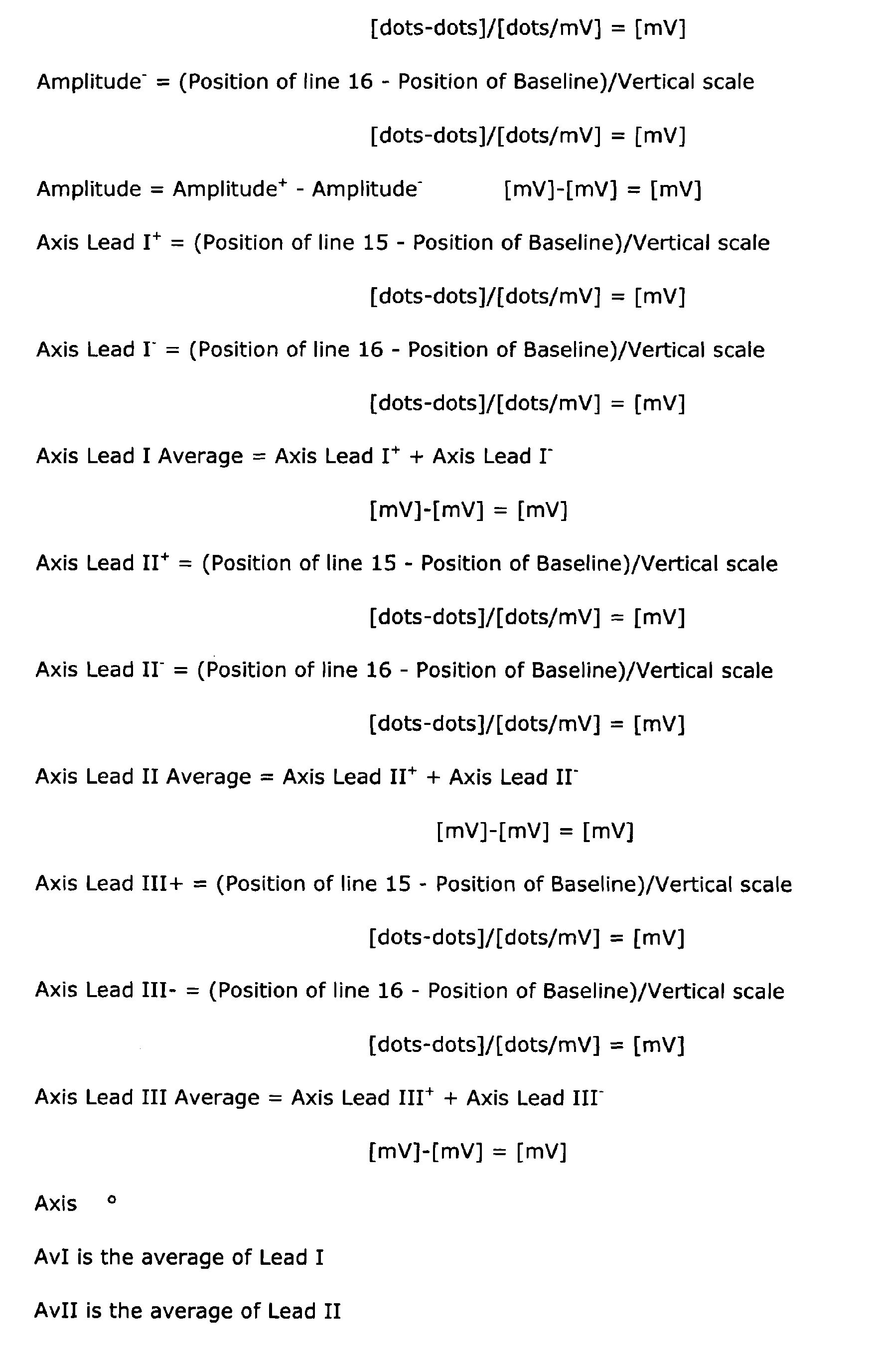

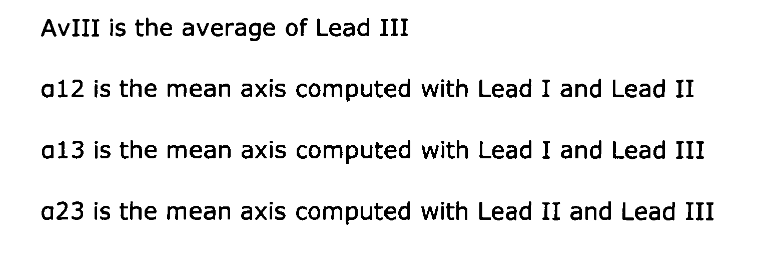

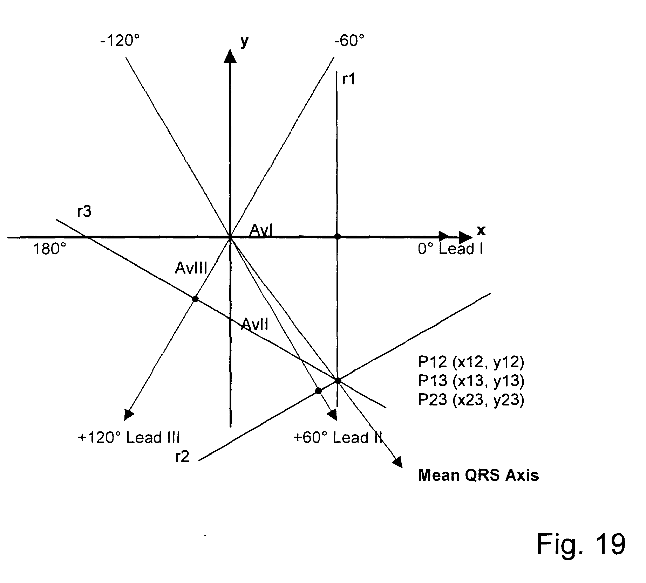

Example shown in Fig. 19:

| | |
|---|---|
| **AvI= 1,350 mV, AvII=** | **2,250 mV, AvIII= 0,900 mV** |
| r1: x=AvI | |
| r2: y=(xsin30°-AvII)/cos30° | |
| x12=AvI | = 1,350 |
| y12=(AvIsin30°-AvII)/cos30° | = -1,819 |
| sinα12=y12/√(x12^2+y12^2) | = -0,803 |
| cosα12=x12/√(x12^2+y12^2) | = 0,596 |
| **α12=arccos(cosα12)*(-sign(sinα12))** | **= 53°** |

| | |
|---|---|
| r1: x=AvI | |
| r3: y=-(xsin30°+AvIII)/cos30° | |
| x13=AvI | = 1,350 |
| y13=-(AvIsin30°+AvIII)/cos30° | = -1,819 |
| sinα13=y13/√(x13^2+y13^2) | = -0,803 |
| cosα13=x13/√(x13^2+y13^2) | = 0,596 |
| **α13=arccos(cosα13)*(-sign(sinα13))** | **= 53°** |

| | |
|---|---|
| r2: y=(xsin30°-AvII)/cos30° | |
| r3: y=-(xsin30°+AvIII)/cos30° | |
| x23=(AvII-AvIII)/(2sin30°) | = 1,350 |
| y23=-(AvII+AvIII)/(2cos30°) | = -1,819 |
| sinα23=y23/√(x23^2+y23^2) | = -0,803 |
| cosα23=x23/√(x23^2+y23^2) | = 0,596 |
| **α23=arccos(cosα23)*(-sign(sina23))** | **= 53°** |
| **αaverage=(α12+α13+α23)/3** | **= 53°** |

## Claims

1. Ruler for evaluation of signals like cardiac signals obtained by ECG, having a template (1) with at least one transparent display (2) adapted to show at least a reference line (12) and a calliper line (13) being parallel to the reference line (12), **characterized in that** at least one input means (4, 5, 6) is provided for setting the distance between the reference line (12) and the calliper line (13) and that the calculation unit is provided for calculating at least one parameter depending on the distance of the reference line (12) to calliper line (13) which parameter is displayed on the display.

2. Ruler of claim 1, **characterized in that** that the display is a LCD-display.

3. Ruler according to one of claim 1 or 2, **characterized in that** that the display is flexible in preferably frameless.

4. Ruler according to claims 1 to 3, **characterized in that** between the reference line (12) and the calliper line (13) a moveable intermediate line (14) is provided.

5. Ruler according to claims 1 to 4, **characterized in that** at least one amplitude line (15, 16) is provided which is parallel to a base line (11) and moveable in vertical direction.

6. Ruler according to claims 1 to 5, **characterized in that** the calculation unit is a micro processor located on the template (1).

7. Ruler according to claims 1 to 6, **characterized in that** the calculation unit and the display are energized by solar cells located on the template (1).

8. Ruler according to claims 1 to 7, **characterized in that** the input means are two switches for increasing and decreasing the distances of different lines and the recording speed.

9. Ruler according to claims 1 to 8, **characterized in that** at least one electrode (9, 10) for obtaining signals and means for displaying the signals on the display (2) is provided.

10. Method for evaluation of signals like cardiac signals obtained by ECG with the following steps:
- obtaining a signal and providing a graphical representation of that signal;
- matching a reference line (12) with a first characteristic point of the graphical representation of the signal;
- matching a caliper line (13) with a second characteristic point of the graphical representation of the signal by acting upon input means;
- conducting a calculation of at least one parameter of the signal in dependency of the distance between reference line (12) and caliper line (13).

11. Method of claim 10, **characterized in that** a transparent display (2) of a ruler is put over the graphical representation of the signal.

12. Method of claim 10, **characterized in that** a ruler contains electrodes with which the signal is obtained.
